(19) Europäisches Patentamt
European Patent Office
Office européen des brevets

(11) Veröffentlichungsnummer: **0 552 760 A1**

(12) # EUROPÄISCHE PATENTANMELDUNG

(21) Anmeldenummer: **93100885.8**

(22) Anmeldetag: **21.01.93**

(51) Int. Cl.5: **C07D 239/30**, C12P 17/12, C12P 7/40, //(C12P17/12, C12R1:40)

(30) Priorität: **24.01.92 CH 200/92**

(43) Veröffentlichungstag der Anmeldung: **28.07.93 Patentblatt 93/30**

(84) Benannte Vertragsstaaten: **AT BE CH DE DK ES FR GB IT LI NL PT SE**

(71) Anmelder: **LONZA AG**

**Gampel/Wallis(CH)**

(72) Erfinder: **Kiener, Andreas, Dr.**
**Meisenweg 5**
**Visp (Kanton Wallis)(CH)**
Erfinder: **Glöcker, Rainer**
**Bifigastrasse**
**Visperterminen (Kanton Wallis)(CH)**
Erfinder: **Stucky, Gerhard, Dr.**
**Spitalweg 9**
**Brig-Glis (Kanton Wallis)(CH)**

(74) Vertreter: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40 (DE)**

(54) **Mikrobiologisches Verfahren zur Herstellung von 2-Halo-pyrimidin-4-carbonsäuren.**

(57) Beschrieben werden neue 2-Halo-pyrimidin-4-carbonsäuren der allgemeinen Formel

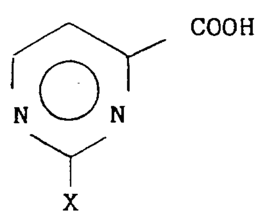

I

sowie ein mikrobiologisches Verfahren zur deren Herstellung, ausgehend von einem 2-Halo-4-methylpyrimidin der allgemeinen Formel

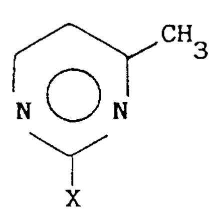

II

Für das Verfahren wird ein Xylol-verwertender Mikroorganismus Pseudomonas putida eingesetzt.

EP 0 552 760 A1

Die Erfindung betrifft neue 2-Halo-pyrimidin-4-carbonsäuren der allgemeinen Formel

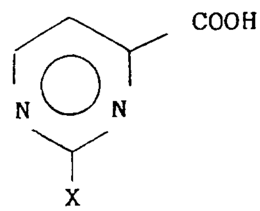

I

worin X ein Halogenatom bedeutet, ein verfahren zu deren Herstellung, ausgehend von einem 2-Halo-4-methylpyrimidin sowie deren Verwendung zur Herstellung von neuen 2-substituierten-pyrimidin-4-carbons äure-derivaten der allgemeinen Formel

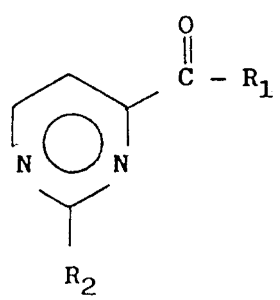

III

worin $R_1$ Halogen, eine $NH_2$-, eine HO-, oder eine $C_1$-$C_4$-Alkoxygruppe und $R_2$ eine $C_1$-$C_4$-Alkoxygruppe oder Halogen bedeutet mit der Ausnahme, dass wenn $R_1$ = eine HO-Gruppe, $R_2$ nicht Halogen bedeutet.

Die erfindungsgemässen 2-Halo-pyrimidin-4-carbonsäuren sind in Form der Säureamide wichtige Zwischenprodukte für pharmazeutische Wirkstoffe (EP 435 749).

Aus dem Stand der Technik ist zwar bekannt, dass Methylgruppen an 5- oder 6-Ring-Heterocyclen mit Mikroorganismen der Gattung Pseudomonas zur entsprechenden Carbonsäure umgesetzt werden (EP-A 442 430).

Es ist jedoch weder ein chemisches noch ein mikrobiologisches Verfahren zur Herstellung der konkreten 2-Halo-pyrimidin-4-carbonsäuren bekannt.

Aufgabe der vorliegenden Erfindung war daher, ein wirtschaftliches und ökologisches mikrobiologisches Verfahren zur Herstellung von neuen 2-Halo-pyrimidin-4-carbonsäuren zur Verfügung zu stellen.

Diese Aufgabe wird mit den neuen 2-Halo-pyrimidin-4-carbonsäuren gemäss Patentanspruch 1 und mit einem neuen Verfahren zu deren Herstellung gemäss Patentanspruch 3 gelöst.

Erfindungsgemäss wird das Verfahren derart durchgeführt, dass man als Substrat ein 2-Halo-4-methylpyrimidin der allgemeinen Formel

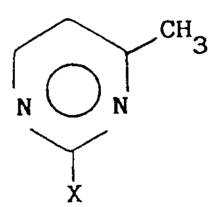

II

worin X die genannte Bedeutung hat mit dem Xylol-verwertenden Mikroorganismus Pseudomonas putida - (DSM 6737) oder dessen Deszendenten und Mutanten in das Endprodukt gemäss Formel I überführt.

Der Mikroorganismus Pseudomonas putida (DSM 6737) wurde am 11.10.1991 bei der Deutschen Sammlung von Mikroorganismen und Zellkulturen GmbH (DSM), Mascheroderweg 1b, 3300 Braunschweig, BRD, hinterlegt.

Als Substrate werden für die Umsetzung käufliche 2-Halo-4-methylpyrimidine der allgemeinen Formel

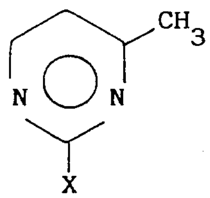

II

worin X ein Halogenatom wie Fluor, Chlor, Brom oder Jod bedeutet, eingesetzt.

Vorzugsweise wird als 2-Halo-4-methylpyrimidin 2-Chlor-4-methylpyrimidin,worin X Chlor bedeutet, angewendet.

Zweckmässig werden die Enzyme des Mikroorganismus vor der eigentlichen Biotransformation mit Xylol oder seinen Isomeren, wie beispielsweise p-Xylol oder m-Xylol bzw. deren Gemische, vorzugsweise mit m-Xylol, induziert.

Die zur Induktion verwendeten Xylole können entweder während der Umsetzung des Substrats anwesend sein oder deren Zufuhr kann vor der Umsetzung des Substrats unterbunden werden.

Die Induktorkonzentration wird üblicherweise so gewählt, dass sie tiefer als die minimale Hemmkonzentration der für die Umsetzung verantwortlichen Enzyme liegt.

Vorzugsweise wird je nach Ausführungsform des Verfahrens die Zufuhr der zur Induktion verwendeten Verbindungen während der Umsetzung des Substrats entweder durch Stoppen der Zufuhr oder durch Abzentrifugieren der Zellen unterbunden.

Der genannte Stamm wächst üblicherweise mit Xylolen, wie p-Xylol, m-Xylol bzw. deren Gemische, vorzugsweise mit m-Xylol als einzige Kohlenstoff und Energiequelle in einem Mineralmedium (Kulla et al., Arch. Microbiol. 135, 1983, S. 1 - 7), in einem Komplexmedium ("Nutrient Broth Nr. 2" Oxoid Ltd., GB) oder in einem Minimalmedium, dessen Zusammensetzung in Tabelle 1 angegeben ist. Das Wachstumssubstrat wird gemäss den Angaben von Claus und Walker (J. Gen. Microbiol. 36, 1964, S. 107 - 122) gasförmig dem Medium zugeführt, wobei die Begasungsrate zweckmässig 0,5 V/min beträgt.

Vor der Substratzugabe werden die Zellen bis zu einer optischen Dichte von 1 bis 200 bei 650 nm im Kulturmedium angezogen, vorzugsweise bis zu einer optischen Dichte von 5 bis 100 bei 650 nm.

Die Umsetzung kann entweder unter einmaliger oder kontinuierlicher Substratzugabe erfolgen, so dass die Substratkonzentration im Kulturmedium 20% (w/v) nicht übersteigt. Vorzugsweise erfolgt die Substratzugabe so, dass die Substratkonzentration im Kulturmedium 5% (w/v) nicht übersteigt.

Die Substratzugabe kann auch, je nach Ausführungsform des Verfahrens, gleichzeitig mit dem Enzyminduktor erfolgen, beispielsweise indem ein Gemisch von Enzyminduktor und Substrat, eingesetzt wird.

Die Umsetzung wird zweckmässig in einem pH-Bereich von 4 bis 11, vorzugsweise von 6 bis 10 durchgeführt.

Zweckmässig wird die Umsetzung bei einer Temperatur von 15 bis 50 °C , vorzugsweise bei einer Temperatur von 25 bis 40 °C durchgeführt.

Die Umsetzung wird üblicherweise in einer Zeit von 1 bis 24 h durchgeführt.

Die bei der Umsetzung gebildeten 2-Halo-pyrimidin-4-carbonsäuren der allgemeinen Formel

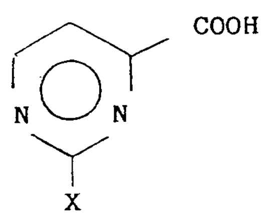

I

worin X ein Halogenatom bedeutet, sind auf chemischem Wege schwer zugänglich und in der Literatur noch nicht beschrieben. Bevorzugte Verbindung ist die 2-Chlor-pyrimidin-4-carbonsäure.

Die Erfindung betrifft ebenfalls die Verwendung der 2-Halo-pyrimidin-4-carbonsäuren der Formel I, zur Herstellung von 2-substituierten-pyrimidin-4-carbonsäure-derivaten der allgemeinen Formel

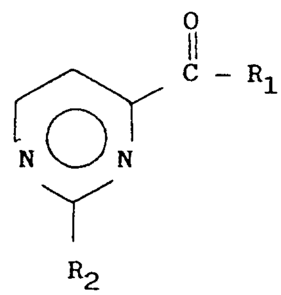

III

worin $R_1$ Halogen, eine $NH_2$-, eine HO- oder eine $C_1$-$C_4$-Alkoxygruppe und $R_2$ eine $C_1$-$C_4$-Alkoxygruppe oder Halogen bedeutet mit der Ausnahme, dass wenn $R_1$ = eine HO-Gruppe, $R_2$ nicht Halogen bedeutet.

Diese 2-substituierten-pyrimidin-4-carbonsäure-derivate der allgemeinen Formel III mit der Ausnahme, dass $R_1$ nicht eine $NH_2$-Gruppe bedeutet, waren ebenfalls bisher chemisch nicht zugänglich und sind damit Bestandteil der Erfindung.

Das Verfahren zu deren Herstellung erfolgt chemisch, ausgehend von den 2-Halo-pyrimidin-4-carbonsäuren der allgemeinen Formel I durch klassische Veresterung, Amidierung, Halogenierung oder Alkoholyse.

Die zweckmässigen Reste $R_1$ und $R_2$ der neuen 2-substituierten-pyrimidin-4-carbonsäure-derivate sind:

$R_1$ = Halogen wie Fluor, Chlor, Brom oder Jod, vorzugsweise Chlor oder $C_1$-$C_4$ Alkoxy- wie z. B. Methoxy-, Ethoxy-, Propoxy- oder Butoxy-, vorzugsweise Methoxy- oder Butoxy- und

$R_2$ = Halogen wie Fluor, Chlor, Brom oder Jod, vorzugsweise Chlor

oder wenn $R_1$ eine HO-Gruppe bedeutet

$R_2$ = $C_1$-$C_4$-Alkoxy- wie beispielsweise Methoxy-, Ethoxy-, Propoxy- oder Butoxy-, vorzugsweise Ethoxy, bedeutet.

Beispiel 1:

Herstellung von 2-Chlor-pyrimidin-4-carbonsäure

Pseudomonas putida (DSM 6737) wurde gemäss EP-A 442 430 mit m-Xylol, als einzige Kohlenstoff- und Energiequelle, in einem 7-Liter Fermenter mit 4,5 l Arbeitsvolumen angezogen. Die Dosierung des Wachstumssubstrates wurde über eine Xylol Messung in der Bioreaktorabluft mit einer Steuerung gekoppelt (EP-P 442 430).

Nachdem eine opt. Dichte bei 650 nm von 20 erreicht war, wurde die Zufuhr des Wachstumssubstrates unterbunden und die Zellsuspension mit 5,0 g (0,04 mol) 2-Chlor-4-methyl-pyrimidin versetzt. Nach 4,5 h konnte mittels Dünnschichtchromatographie kein Ausgangsmaterial mehr nachgewiesen werden. 2-Chlor-pyrimidin-4-carbonsäure wurde nicht weiter metabolisiert. Die zellfreie Fermentationslösung wurde bis auf 100 ml eingeengt und der pH mit $H_2SO_4$ auf 2,0 eingestellt und fünfmal mit je 100 ml Ethylacetat extrahiert. Die organische Phase wurde bis zur Trockene eingeengt und der Rückstand zweimal aus Wasser umkristallisiert. Es konnten 2,2 g (0,014 mol) 2-Chlor-pyrimidin4-carbonsäure entsprechend einer Ausbeute von 28%, bez. eingesetztes 2-Chlor-4-methlypyrimidin, erhalten werden.

$^1$H-NMR: (DMSO-$d^6$, 300 MHz) $\delta$ in ppm 2,5, s;
8,05, d;
9,05, d.

Tabelle 1

| | | |
|---|---|---|
| **Mediumzusammensetzung:** | | |
| - $MgCl_2 \cdot 6H_2O$ | 0,8 | g/l |
| - $CaCl_2$ | 0,16 | g/l |
| - $(NH_4)SO_4$ | 2 | g/l |
| - $NH_4Cl$ | 5 | g/l |
| - $Na_2SO_4$ | 0,25 | g/l |
| - $KH_2SO_4$ | 0,4 | g/l |
| - $Na_2HPO_4$ | 0,9 | g/l |
| - Spurenelemente | 1 | ml/l |
| - FeEDTA | 15 | ml/l |
| **Zusammensetzung der Spurenelementlösung:** | | |
| - KOH | 15 | g/l |
| - $EDTANa_2 \cdot 2H_2O$ | 10 | g/l |
| - $ZnSO_4 \cdot 7H_2O$ | 9 | g/l |
| - $MnCl_2 \cdot 4H_2O$ | 4 | g/l |
| - $H_3BO_3$ | 2,7 | g/l |
| - $CoCl_2 \cdot 6H_2O$ | 1,8 | g/l |
| - $CuCl_2 \cdot 2H_2O$ | 1,5 | g/l |
| - $NiCl_2 \cdot 6H_2O$ | 0,18 | g/l |
| - $Na_2MoO_4 \cdot 2H_2O$ | 0,2 | g/l |
| **Zusammensetzung von FeEDTA:** | | |
| - $EDTA\ Na_2 \cdot 2H_2O$ | 5 | g/l |
| - $FeSO_4 \cdot 7H_2O$ | 2 | g/l |

Beispiel 2:

Herstellung von 2-Chlorpyrimidin-4-carbonsäurechlorid

Zu einer Lösung von 3,0 g (18,9 mmol) 2-Chlorpyrimidin-4-carbonsäure in 60 ml Thionylchlorid wurden 12 Tropfen Dimethylformamid zugegeben. Die Reaktion wurde anschliessend während 6 h am Rückfluss erhitzt. Nach Abkühlen auf Raumtemperatur wurde überschüssiges Thionylchlorid am Rotationsverdampfer entfernt und der Rückstand destilliert (Sdp. ca. 100 °C/1 mbar). Man erhielt 2,95 g Produkt, entsprechend einer Ausbeute von 88%, bez. eingesetzter 2-Chlorpyrimidin-4-carbonsäure, welches in der Vorlage kristallisierte.

Schmelzpunkt: 52,2 - 53,1 °C

$^1H$-NMR: ($CDCl_3$, 300 MHz) $\delta$ in ppm 7,25, s; 7,95, d; 9,0, d.

Beispiel 3:

Herstellung von 2-Chlorpyrimidin-4-carbonsäuremethylester

Eine Lösung von 0,5 g (2,82 mmol) 2-Chlorpyrimidin-4-carbonsäurechlorid in 10 ml Methanol wurde 45 min bei Raumtemperatur gerührt. Anschliessend wurde die Reaktionslösung auf 80 ml gesättigte $NaHCO_3$-Lösung gegossen und 3mal mit je 50 ml Ethylacetat extrahiert. Die gemeinsame organische Phase wurde mit 60 ml gesättigter NaCl-Lösung gewaschen, über $Na_2SO_4$ getrocknet und am Rotationsverdampfer eingeengt. Nach Trocknen am Hochvakuum erhielt man 0,50 g Produkt als weissen Feststoff, entsprechend einer Ausbeute von 100%, bez. eingesetztem 2-Chlorpyrimidin-4-carbonsäurechlorid.
Schmelzpunkt: 93,6 - 96,6 °C.
$^1$H-NMR: (DMSO, 300 MHz) $\delta$ in ppm 2,5, s; 3,95, s; 8,1, d; 9,1, d.

Beispiel 4:

Herstellung von 2-Chlorpyrimidin-4-carbonsäurebutylester

Eine Lösung von 0,52 g (2,93 mmol) 2-Chlorpyrimidin-4-carbonsäurechlorid in 10 ml Butanol wurde 1,5 h bei Raumtemperatur gerührt. Anschliessend wurde die Reaktionslösung auf 80 ml $NaHCO_3$-Lösung gegossen und 3mal mit je 60 ml Ethylacetat extrahiert. Die gemeinsame organische Phase wurde mit 60 ml gesättigter NaCl-Lösung gewaschen, über $Na_2SO_4$ getrocknet und am Rotationsverdampfer eingeengt. Nach Trocknen des Produktes am Hochvakuum erhielt man 0,53 g Produkt als weissen Feststoff, entsprechend einer Ausbeute von 85%, bez. eingesetztem 2-Chlorpyrimidin-4-carbonsäurechlorid.
Schmelzpunkt: 67,9 - 68,8 °C.
$^1$H-NMR: ($CDCl_3$, 300 MHz) $\delta$ in ppm 1,0, t; 1,5, m; 1,8, m; 4,5, t; 7,25, s; 7,55, d; 8,9, d.

Beispiel 5:

Herstellung von 2-Chlorpyrimidin-4-carboxamid

Zu 20 ml Tetrahydrofuran wurde bei -6 °C während 35 min Ammoniak-Gas eingeleitet. Anschliessend wurde auf 10 °C erwärmt und 1,5 g (8,47 mmol) 2-Chlorpyrimidin-4-carbonsäurechlorid zugegeben. Die Reaktionslösung wurde während 45 min bei Raumtemperatur gerührt. Anschliessend wurde am Rotationsverdampfer eingeengt, der Rückstand auf 90 ml gesättigte $NaHCO_3$-Lösung gegossen und die Wasserphase 3mal mit je 50 ml Ethylacetat extrahiert. Die gemeinsame organische Phase wurde mit gesättigter NaCl-Lösung gewaschen, über $Na_2SO_4$ getrocknet und am Rotationsverdampfer eingeengt. Nach Trocknen am Hochvakuum erhielt man 1,07 g Produkt als weissen Feststoff, entsprechend einer Ausbeute von 80%, bez. eingesetztem 2-Chlorpyrimidin-4-carbonsäurechlorid.
Schmelzpunkt: 147,2 - 151,4 °C.
$^1$H-NMR: ($CDCl_3$, 300 MHz) $\delta$ in ppm 6,2, br.s; 7,25, s; 7,7, br.s; 8,1, d; 8,9, d.

Beispiel 6:

Herstellung von 2-Ethoxypyrimidin-4-carbonsäure

Eine Suspension von 1,6 g (10 mmol) 2-Chlorpyrimidin-4-carbonsäure in 40 ml 0,75 molarer Natriumethanolatlösung in Ethanol wurde während 4,5 h am Rückfluss erhitzt. Man liess auf 40 °C abkühlen und zur Reaktionslösung wurden 80 ml einer 1normalen HCl-Lösung zugegeben. Die Wasserphase wurde 3mal mit je 60 ml Ethylacetat extrahiert, die gemeinsame organische Phase mit $Na_2SO_4$ getrocknet und am Rotationsverdampfer eingeengt. Nach Trocknen am HV erhielt man 1,55 g Produkt als leicht braunes Pulver, entsprechend einer Ausbeute von 91%, bez. eingesetzter 2-Chlorpyrimidin-4-carbonsäure.
Schmelzpunkt: 177,4 - 178,1 °C.
$^1$H-NMR: ($CDCl_3$, 300 MHz) $\delta$ in ppm 1,5, t; 4,5, m; 7,25, s; 7,75 d; 8,85, d.

## Patentansprüche

**1.** 2-Halo-pyrimidin-4-carbonsäuren der allgemeinen Formel

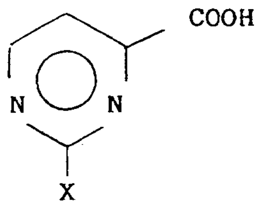

I

worin X ein Halogenatom bedeutet.

**2.** 2-Chlor-pyrimidin-4-carbonsäure.

**3.** Verfahren zur Herstellung von 2-Halo-pyrimidin-4-carbonsäuren der allgemeinen Formel

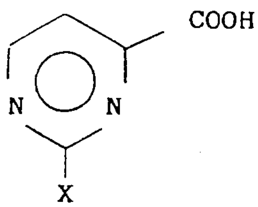

I

worin X die genannte Bedeutung hat, dadurch gekennzeichnet, dass man als Substrat ein 2-Halo-4-methylpyrimidin der allgemeinen Formel

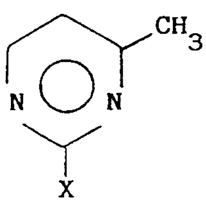

II

worin X die genannte Bedeutung hat, mit dem Xylol-verwertenden Mikroorganismus Pseudomonas putida oder dessen Deszendenten und Mutanten (DSM 6737) in das Endprodukt gemäss Formel I überführt.

**4.** Verfahren nach Patentanspruch 3, dadurch gekennzeichnet, dass man als 2-Halo-4-methylpyrimidin der Formel II 2-Chlor-4-methylpyrimidin anwendet, worin X Chlor bedeutet.

**5.** Verfahren nach mindestens einem der Patentansprüche 3 und 4, dadurch gekennzeichnet, dass die Enzyme des Mikroorganismus Pseudomonas putida (DSM 6737) mit Xylol induziert werden.

**6.** Verfahren nach mindestens einem der Patentansprüche 3 bis 5, dadurch gekennzeichnet, dass die Umsetzung unter einmaliger oder kontinuierlicher Substratzugabe durchgeführt wird, so dass die Substratkonzentration im Kulturmedium 20% (w/v) nicht übersteigt.

**7.** Verfahren nach mindestens einem der Patentansprüche 3 bis 6, dadurch gekennzeichnet, dass die Umsetzung bei einem pH von 4 bis 11 und bei einer Temperatur von 15 bis 50 ° C durchgeführt wird.

**8.** Verwendung der 2-Halo-pyrimidin-4-carbonsäuren der allgemeinen Formel

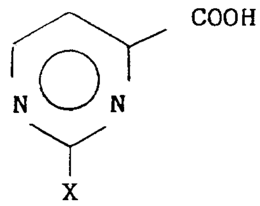

I

worin X die genannte Bedeutung hat, zur Herstellung, auf chemischem Weg, von 2-substituierten-pyrimidin-4-carbonsäure-derivaten der allgemeinen Formel

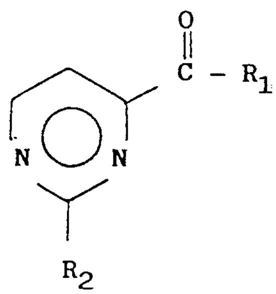

III

worin $R_1$ Halogen, eine $NH_2$-, eine HO- oder eine $C_1$-$C_4$-Alkoxygruppe und $R_2$ eine $C_1$-$C_4$-Alkoxygruppe oder Halogen bedeutet mit der Ausnahme, dass wenn $R_1$ = eine HO-Gruppe, $R_2$ nicht Halogen bedeutet.

**9.** 2-substituierte-pyrimidin-4-carbonsäure-derivate der allgemeinen Formel

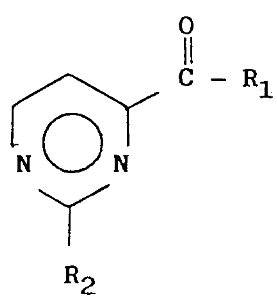

III

worin $R_1$ und $R_2$ die genannte Bedeutung haben mit der Ausnahme, dass $R_1$ nicht eine $NH_2$-Gruppe bedeutet.

**10.** 2-substituierte-pyrimidn-4-carbonsäure-derivate nach Anspruch 9 worin $R_1$ Chlor, eine Methoxy- oder Butoxygruppe und $R_2$ Chlor bedeutet.

**11.** 2-substituierte-pyrimidin-4-carbonsäure-derivate nach Anspruch 9 worin $R_1$ eine HO-Gruppe und $R_2$ eine Ethoxygruppe bedeutet.

Europäisches Patentamt

# EUROPÄISCHER RECHERCHENBERICHT

Nummer der Anmeldung

EP 93 10 0885

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 466 042 (LONZA AG)<br>* das ganze Dokument *<br>--- | 1,3 | C07D239/30<br>C12P17/12<br>C12P7/40<br>//(C12P17/12,<br>C12R1:40) |
| A,D | EP-A-0 442 430 (LONZA AG)<br>* das ganze Dokument *<br>--- | 1,3 | |
| A,D | EP-A-0 435 749 (SYNTHELABO)<br>* Seite 5; Abbildung VI *<br>--- | 9 | |
| X | CHEMICAL ABSTRACTS, vol. 94, no. 1,<br>5. Januar 1981, Columbus, Ohio, US;<br>abstract no. 3979c,<br>WARCZYKOWSKA IWONA ET AL. 'Synthesis of new pyrimidine-4-carboxylic acid derivatives'<br>Seite 372 ;Spalte 1 ;<br>* Zusammenfassung *<br>& POL. J. CHEM.<br>Bd. 54, Nr. 2, 1980, POLAND<br>Seiten 335 - 340<br>----- | 9 | |
| | | | RECHERCHIERTE SACHGEBIETE (Int. Cl.5) |
| | | | C07D<br>C12P |

Der vorliegende Recherchenbericht wurde für alle Patentansprüche erstellt

| Recherchenort | Abschlußdatum der Recherche | Prüfer |
|---|---|---|
| BERLIN | 30 APRIL 1993 | KYRIAKAKOU G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)